# EUROPÄISCHE PATENTSCHRIFT

(11) **EP 2 155 660 B1**
(45) Veröffentlichungstag und Bekanntmachung des Hinweises auf die Patenterteilung: **27.02.2013**
(21) Anmeldenummer: 08736313.1
(22) Anmeldetag: 17.04.2008
(51) Int. Cl.: C07C 227/42

(54) **Verfahren zur Kristallisation von 2-(4-N,N-diethylamino-2-hydroxybenzoyl)-benzoesäure-N-hexylester**
Method for the crystallization of 2-(4-N,N-diethyl amino-2-hydroxy benzoyl)-benzoic acid-N-hexyl ester
Procédé de cristallisation de l'ester N-hexylique de l'acide 2-(4-N,N-diéthylamino-2-hydroxybenzoyl)-benzoïque

(30) Priorität: 02.05.2007 EP 07107342
(43) Veröffentlichungstag der Anmeldung: 24.02.2010
(73) Patentinhaber: BASF SE, 67056 Ludwigshafen (DE)
(72) Erfinder: CHAMP, Samantha, 67063 Ludwigshafen (DE); HETTERICH, Manfred, 67127 Rödersheim-Gronau (DE); GOTTWALD, Günther, 68167 Mannheim (DE)
(86) Internationale Anmeldenummer: PCT/EP2008/054645
(87) Internationale Veröffentlichungsnummer: WO 2008/135360

(56) Entgegenhaltungen:
- WO-A-03/097578

## Beschreibung

Die vorliegende Erfindung betrifft ein Verfahren zur Kristallisation von 2-(4-N,N-Diethylamino-2-hydroxybenzoyl)-benzoesäure-n-hexylester, ein Verfahren zur Herstellung von rieselfähigen oder schüttbaren Teilchen von kristallinem 2-(4-N,N-Diethylamino-2-hydroxybenzoyl)-benzoesäure-n-hexylester und bestimmten rieselfähigen oder schüttbaren Teilchen von kristallinem 2-(4-N,N-Diethylamino-2-hydroxybenzoyl)-benzoesäure-n-hexylester.

Die Auswirkungen des Sonnenlichtes, insbesondere der UV-B- und der UV-A-Strahlung auf die menschliche Haut und die daraus resultierenden Hautreaktionen, sowie die Möglichkeiten zum Schutz der Haut sind zu einem Großteil schon bekannt und werden im Detail weiter untersucht (Chemie in unserer Zeit, 2004, 38, 98-112).

Während die UV-B Strahlung (290-320 nm) insbesondere für die Entstehung von Sonnenbrand verantwortlich ist, führt die UV-A Strahlung zur sogenannten vorzeitigen Hautalterung durch Schädigung der Collagen- und Elastinfasern. Weiterhin ist die UV-A Strahlung in der Lage, die DNA zu schädigen, was im schlimmsten Fall zu Hautkrebs führen kann.

Die Industrie bietet daher sowohl sogenannte UV-B-Filter als auch UV-A-Filter an, um die schädigenden Wirkungen der Sonnenstrahlung auf die menschliche Haut zu vermindern.

EP-A-1 046 391 beschreibt die Verwendung von aminosubstituierten Hydroxybenzophenonen als photostabile UV-A-Filter in kosmetischen Zubereitungen.

In WO 03/097578 wird ein Verfahren zur Herstellung von 2-(4-N,N-Diethylamino-2-hydroxybenzoyl)-benzoesäure-n-hexylester der Formel I 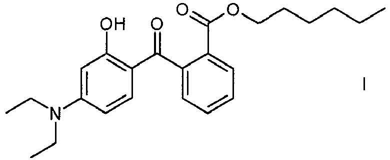 beschrieben, wobei das in der Synthese aus Lösung kristallin anfallende rosa gefärbte Rohprodukt zunächst chromatographisch gereinigt und anschließend destillativ von den vorhandenen Lösungsmitteln befreit wird. Zum Schluss wird das saubere Endprodukt als Schmelze abgefüllt. Der entstandene Ester kann zusätzlich auch aus Cyclohexan bei 10°C zur Kristallisation gebracht werden.

2-(4-N,N-Diethylamino-2-hydroxybenzoyl)-benzoesäure-n-hexylester wird kommerziell von der BASF Aktiengesellschaft unter der Warenbezeichnung Uvinul^{®} A Plus als UV-A-Filter vermarktet. Da das Produkt als Schmelze abgefüllt wird und nach etwa sechs Wochen Lagerung bei Raumtemperatur erstes Kristallwachstum auftritt, muss der Anwender das gesamte Gebinde auf eine Temperatur über dem Schmelzpunkt von 2-(4-N,N-Diethylamino-2-hydroxybenzoyl)-benzoesäure-n-hexylester erwärmen, um anschließend flüssiges Produkt aus dem Gebinde entnehmen zu können.

In WO 2005/025529 wird eine pulverförmige Zubereitung von 2-(4-N,N-Diethylamino-2-hydroxybenzoyl)-benzoesäure-n-hexylester (Uvinul^{®} A Plus) beschrieben, wobei der UV-A-Filter in modifizierter Stärke als Schutzkolloid eingebettet ist. Das Trockenpulver lässt sich erneut in Wasser redispergieren, wobei der UV-A-Filter in kolloiddisperser Form vorliegt. Das Schutzkolloid muss in den späteren Anwendungen toleriert werden.

Es war Aufgabe der vorliegenden Erfindung, ein einfaches und wirtschaftliches Verfahren zur Kristallisation von 2-(4-N,N-Diethylamino-2-hydroxybenzoyl)-benzoesäure-n-hexylester sowie ein effizientes Verfahren zur Herstellung von rieselfähigen oder schüttbaren Teilchen von kristallinem 2-(4-N,N-Diethylamino-2-hydroxybenzoyl)-benzoesäure-n-hexylester bereitzustellen. Weiterhin sollte eine vom Verarbeiter sicher und einfach handhabbare Form von kristallinem 2-(4-N,N-Diethylamino-2-hydroxybenzoyl)-benzoesäure-n-hexylester bereitgestellt werden.

Diese Aufgabe wird durch ein Verfahren zur Kristallisation von 2-(4-N,N-Diethylamino-2-hydroxybenzoyl)-benzoesäure-n-hexylester, umfassend die Verfahrensschritte
a) Bereitstellung einer klaren Schmelze von 2-(4-N,N-Diethylamino-2-hydroxybenzoyl)-benzoesäure-n-hexylester bei einer Temperatur oberhalb von 57°C, und
b) Auskristallisation von 2-(4-N,N-Diethylamino-2-hydroxybenzoyl)-benzoesäure-n-hexylester bei einer Temperatur unterhalb von 57°C,
dadurch gekennzeichnet, dass die klare Schmelze aus Verfahrensschritt a) bei einer Temperatur unterhalb von 57°C so lange gerührt wird, bis eine Trübung auftritt, bevor Verfahrensschritt b) ausgeführt wird,
gelöst.
der Synthese wie Ausgangsprodukte, Zwischenprodukte, Nebenprodukte und/oder Lösungsmittel enthalten.

Der Gehalt von 2-(4-N,N-Diethylamino-2-hydroxybenzoyl)-benzoesäure-n-hexylester in der in Verfahrensschritt a) bereitgestellten Schmelze beträgt bevorzugt mindestens 90 Gew.-%, besonders bevorzugt mindestens 95 Gew.-%, insbesondere mindestens 98 Gew.-%.

Bevorzugt wird demnach ein erfindungsgemäßes Verfahren, in dem die Schmelze von 2-(4-N,N-Diethylamino-2-hydroxybenzoyl)-benzoesäure-n-hexylester in Verfahrensschritt a) eine Reinheit von mindestens 98 Gew.-%, bevorzugt mindestens 98,5 Gew.-%, insbesondere mindestens 99 Gew.-% aufweist.

Da 2-(4-N,N-Diethylamino-2-hydroxybenzoyl)-benzoesäure-n-hexylester bei etwa 57°C schmilzt, reicht es prinzipiell aus, das feste Ausgangsmaterial einer Temperatur von nur wenig oberhalb von 57°C auszusetzen. Üblicherweise wird eine klare Schmelze bei einer Temperatur von 57 bis 80°C, bevorzugt von 58 bis 65°C, insbesondere von 59 bis 62°C bereitgestellt.

Da die Kristallisation von 2-(4-N,N-Diethylamino-2-hydroxybenzoyl)-benzoesäure-n-hexylester nicht spontan beim Schmelzpunkt der Verbindung von 57°C einsetzt, wird wie eingangs beschrieben unterhalb des Schmelzpunktes, beispielsweise bei Raumtemperatur eine thermodynamisch metastabile Schmelze gebildet.

In Verfahrensschritt b) des erfindungsgemäßen Verfahrens wird 2-(4-N,N-Diethylamino-2-hydroxybenzoyl)-benzoesäure-n-hexylester bei einer Temperatur unterhalb von 57°C auskristallisiert, indem die klare Schmelze aus Verfahrensschritt a) bei einer Temperatur unterhalb von 57°C so lange gerührt wird, bis eine Trübung auftritt, bevor Verfahrensschritt b) ausgeführt wird. Bevorzugt wird die in Verfahrensschritt a) hergestellte Schmelze bei einer Temperatur zwischen 25 und 40°C, besonders bevorzugt bei einer Temperatur zwischen 27 und 35°C bis zur Trübung gerührt.

Prinzipiell kann zum Rühren der Schmelze unterhalb von 57°C jeder Rührertyp eingesetzt werden, wie beispielsweise ein Magnetrührkern, ein Ankerrührer, ein Propellerrührer, ein Schrägblattrührer oder ein Scheibenrührer. Die Größe des Rührers im Verhältnis zum Volumen der Schmelze ist im Grunde genommen nicht entscheidend. Bevorzugt homogenisiert der Rührer die gesamte Schmelze während der Rührzeit, insbesondere wenn die Schmelze zum Kristallisieren portionsweise in verschiedene Gefäße umgefüllte wird.

Es wurde festgestellt, dass die Zeit bis zum Auftreten einer Trübung in der Schmelze, die durch die Bildung von Kristallkeimen hervorgerufen wird, verringert werden kann, wenn die Geschwindigkeit des Rührers, das heißt die Umdrehungszahl des Rührers pro Zeiteinheit erhöht wird. Bevorzugt wird in dem erfindungsgemäßen Verfahren die Schmelze mit einem Rührer bei einer Geschwindigkeit von 100 bis 600 U/min, besonders bevorzugt bei einer Geschwindigkeit von 200 bis 500 U/min gerührt.

In dem erfindungsgemäßen Verfahren wird bevorzugt die Schmelze mit einem Rührer, insbesondere einem Propellerrührer, der einen Durchmesser von 2 bis 20 cm aufweist, bei 200 bis 500 U/min, insbesondere 300 bis 400 U/min gerührt.

In dem erfindungsgemäßen Verfahren zur Kristallisation von 2-(4-N,N-Diethylamino-2-hydroxybenzoyl)-benzoesäure-n-hexylester wird Verfahrensschritt b) bevorzugt ohne Rühren durchgeführt, da sich die durch Kristallisation verfestigende Schmelze ohnehin ab einem gewissen Zeitpunkt nicht mehr rühren lässt. Daher kann nach dem Auftreten der Trübung die noch flüssige Schmelze in eine gewünschte Form gefüllt werden, worin dann der Auskristallisationsschritt stattfindet. Prinzipiell sind die Geometrie und der Aufbau der Form ohne Bedeutung. Bevorzugt werden jedoch solche Formen verwendet, bei denen die Entnahme des festen, auskristallisierten Esters keine Schwierigkeiten bereitet. Konische Formen haben sich als vorteilhaft herausgestellt. Das Volumen der Formen ist im Prinzip beliebig und kann beispielsweise von 0,1 ml bis 10 l oder auch 5 l bis 100 l varieren. Das aus der Form entnommene Produkt kann wiederum in geeigneten Vorrichtungen, wie beispielsweise einer Mühle, weiter zerkleinert werden.

In dem erfindungsgemäßen Verfahren kann in Verfahrensschritt b) als Form auch eine ebene Fläche eingesetzt werden, auf welche die noch flüssige Schmelze des Esters als eine dünne Schicht, d. h. mit einer geringen Dicke im Vergleich zur Längen-BreitenAusdehnung , bevorzugt zwischen 0,1 und 5 mm Dicke, insbesondere zwischen 0,2 und 2 mm Dicke, aufgetragen wird. Alternativ kann die noch flüssige Schmelze auch in kleinen Tropfen portioniert auf die ebene Fläche gegeben werden, so dass sich so genannte Pastillen oder Prills, halbkugelförmige Gebilde, ausformen. Bevorzugt liegt der Durchmesser der Halbkugeln zwischen 0,1 und 5 mm. Nach dem Auskristallisieren des Esters werden die feste Schicht oder die festen Pastillen oder Prills für gewöhnlich von der Ebene entfernt und abgefüllt, wobei üblicherweise die dünnen Schichten auf eine gewünschte Schuppengröße durch Zerbrechen zerkleinert werden. Der Herstellungsprozess von Schuppen, Pastillen und Prills kann diskontinuierlich (Batchprozess) oder kontinuierlich erfolgen, wobei in einem kontinuierlichen Verfahren beispielsweise ein kontinuierlich umlaufendes Stahlband als Form im Sinne der vorliegenden Erfindung eingesetzt werden kann.

Ein weiterer Gegenstand der vorliegenden Erfindung ist ein Verfahren zur Herstellung von rieselfähigen oder schüttbaren Teilchen von kristallinem 2-(4-N,N-Diethylamino-2-hydroxybenzoyl)-benzoesäure-n-hexylester mit einem mittleren Teilchendurchmesser von 10 µm bis 22 cm, umfassend die Schritte
a') Bereitstellung einer klaren Schmelze von 2-(4-N,N-Diethylamino-2-hydroxybenzoyl)-benzoesäure-n-hexylester bei einer Temperatur oberhalb von 57°C,
b') Rühren der Schmelze aus Verfahrensschritt a') bei einer Temperatur unterhalb von 57°C bis eine Trübung auftritt,
c') Abfüllen der trüben Schmelze in eine Form,
d') Auskristallisation der abgefüllten Schmelze von 2-(4-N,N-Diethylamino-2-hydroxybenzoyl)-benzoesäure-n-hexylester bei einer Temperatur unterhalb von 57°C in der Form,
e') Entnahme des auskristallisierten 2-(4-N,N-Diethylamino-2-hydroxybenzoyl)-benzoesäure-n-hexylesters aus der Form und optional
f') Zerkleinern des kristallinen 2-(4-N,N-Diethylamino-2-hydroxybenzoyl)-benzoesäure-n-hexylesters aus Verfahrensschritt e') auf die gewünschte Teilchengröße.

Nach dem erfindungsgemäßen Verfahren lassen sich rieselfähige oder schüttbare Teilchen von kristallinem 2-(4-N,N-Diethylamino-2-hydroxybenzoyl)-benzoesäure-n-hexylester mit einem mittleren Teilchendurchmesser von 10 µm bis 22 cm herstellen.

Die Größe der Teilchen kann durch die Wahl der Form, in die die trübe Schmelze gefüllt wird, oder durch einen weiteren Zerkleinerungsschritt beliebig eingestellt werden. Bevorzugt werden nach dem erfindungsgemäßen Verfahren Teilchen von kristallinem 2-(4-N,N-Diethylamino-2-hydroxybenzoyl)-benzoesäure-n-hexylester mit einem mittleren Teilchendurchmesser von 0,1 bis 5 mm hergestellt.

Wie voranstehend bereits beschrieben, kann der 2-(4-N,N-Diethylamino-2-hydroxybenzoyl)-benzoesäure-n-hexylester bedingt durch seine Herkunft noch geringe Mengen Verunreinigungen aus der Synthese wie Ausgangsprodukte, Zwischenprodukte, Nebenprodukte und/oder Lösungsmittel enthalten.

Der Gehalt von 2-(4-N,N-Diethylamino-2-hydroxybenzoyl)-benzoesäure-n-hexylester in der in Verfahrensschritt a') bereitgestellten Schmelze beträgt bevorzugt mindestens 90 Gew.-%, besonders bevorzugt mindestens 95 Gew.-%, insbesondere mindestens 98 Gew.-%.

Bevorzugt wird demnach ein erfindungsgemäßes Verfahren, in dem die Schmelze von 2-(4-N,N-Diethylamino-2-hydroxybenzoyl)-benzoesäure-n-hexylester in Verfahrensschritt a') eine Reinheit von mindestens 98 Gew.-%, bevorzugt mindestens 98,5 Gew.-%, insbesondere mindestens 99 Gew.-% aufweist.

In Verfahrensschritt b') des erfindungsgemäßen Verfahrens wird die Schmelze aus Verfahrensschritt a') bei einer Temperatur unterhalb von 57°C gerührt, bis eine Trübung auftritt. Bevorzugt wird Verfahrensschritt b') bei einer Temperatur zwischen 25 und 40°C, besonders bevorzugt bei einer Temperatur zwischen 27 und 35°C durchgeführt.

Bezüglich der in Verfahrensschritt b') verwendbaren Rührer und Rührbedingungen, sowie bevorzugten Ausführungsformen wird auf die vorangehenden Ausführungen verwiesen.

Die auftretende Trübung kann visuell durch in Augenscheinnahme wahrgenommen werden oder aber auch mittels einer oder mehrerer geeigneter Messzellen ermittelt werden, die sich in der Schmelze befinden. Es wurde weiterhin festgestellt, dass das Auftreten der Trübung einhergeht mit einer Verlangsamung der Abkühlgeschwindigkeit bis hin zu einem leichten Temperaturanstieg ohne externe Energiezufuhr bedingt durch das Freiwerden von Kristallisationswärme. In den Versuchen zeigte es sich, dass bei Erreichen eines solchen Temperaturzustandes der Schmelze diese trüb war, sich noch problemlos in eine Form umfüllen ließ und anschließend spätestens nach 2 Tagen vollständig durchkristallisiert war.

In Verfahrensschritt c') wird die trübe Schmelze in eine Form abgefüllt, wobei prinzipiell die Geometrie und der Aufbau der Form ohne Bedeutung sind. Bevorzugt werden jedoch solche Formen verwendet, bei denen die Entnahme des festen, auskristallisierten Esters keine Schwierigkeiten bereitet. Konische Formen haben sich als vorteilhaft herausgestellt. Das Volumen der Formen ist im Prinzip beliebig und kann beispielsweise von 0,1 ml bis 10 l oder auch 5 l bis 100 l varieren. Ebenfalls vorteilhaft sind die vorangehend beschriebenen ebenen Flächen als Form, von denen der Ester ebenfalls problemlos nach dem Auskristallisieren abgetrennt werden kann.

In Verfahrensschritt d') lässt man die in die Form abgefüllte oder auf die ebene Fläche als Form aufgegebene Schmelze von 2-(4-N,N-Diethylamino-2-hydroxybenzoyl)-benzoesäure-n-hexylester bei einer Temperatur unterhalb von 57°C auskristallisieren. Bevorzugt lässt man die Schmelze bei einer Temperatur von 15 bis 35°C auskristallisieren.

In Verfahrensschritt e') wird der auskristallisierte 2-(4-N,N-Diethylamino-2-hydroxybenzoyl)-benzoesäure-n-hexylester aus der Form entnommen und optional wird der kristalline 2-(4-N,N-Diethylamino-2-hydroxybenzoyl)-benzoesäure-n-hexylester auf die gewünschte Teilchengröße zerkleinert. Bevorzugt ist ein Verfahren, in dem das verfestigte Produkt aus Verfahrensschritt e') zerkleinert wird, bevorzugt auf eine mittlere Teilchengröße von 0,1 bis 5 mm. Wurde in Verfahrensschritt c') eine ebene Fläche als Form in Sinne der vorliegenden Erfindung verwendet, beispielsweise ein Stahlband, so kann das in Schichten anfallende Produkt nach Trennung von der ebenen Fläche durch Zerbrechen zerkleinert werden, während das direkt in der gewünschten Größe als Pastillen oder Prills anfallende Produkt nach der Trennung von der ebenen Fläche üblicherweise nicht weiter zerkleinert werden wird.

Der Zerkleinerungsschritt f) kann mit verschiedenen Mitteln oder in geeigneten Vorrichtungen durchgeführt werden. Bevorzugt wird der Zerkleinerungsschritt in einer Mühle durchgeführt, wobei sich die Wahl der Mühle nach dem gewünschten Grad der Zerkleinerung richtet.

Ein weiterer Gegenstand der vorliegenden Erfindung sind rieselfähige oder schüttbare Teilchen von kristallinem 2-(4-N,N-Diethylamino-2-hydroxybenzoyl)-benzoesäure-n-hexylester mit einem mittleren Teilchendurchmesser von 0,1 bis 5 mm, einer Schüttdichte größer als 0,35 g/ml, bevorzugt größer als 0,4 g/ml, insbesondere größer als 0,5 g/ml und einer Reinheit von mindestens 98 Gew.-%, bevorzugt mindestens 98,5 Gew.-%, insbesondere von mindestens 99 Gew.-%.

Die vorliegende Erfindung betrifft auch die Verwendung der oben beschriebenen rieselfähigen oder schüttbaren Teilchen von kristallinem 2-(4-N,N-Diethylamino-2-hydroxybenzoyl)-benzoesäure-n-hexylester mit einem mittleren Teilchendurchmesser von 0,1 bis 5 mm, einer Schüttdichte größer als 0,35 g/ml, bevorzugt größer als 0,4 g/ml, insbesondere größer als 0,5 g/ml und einer Reinheit von mindestens 98 Gew.-%, bevorzugt mindestens 98,5 Gew.-%, insbesondere von mindestens 99 Gew.-% als UV-Filter oder als Radikalfänger in kosmetischen und dermatologischen Zubereitungen oder als Produktschutz.

Der Vorteil des erfindungsgemäßen Verfahren beruht darin, den kristallinen 2-(4-N,N-Diethylamino-2-hydroxybenzoyl)-benzoesäure-n-hexylester reproduzierbar und zeitsparend ausgehend von einer metastabilen Schmelze des hochreinen Esters herstellen zu können. Die erfindungsgemäßen rieselfähigen oder schüttbaren Teilchen von kristallinem 2-(4-N,N-Diethylamino-2-hydroxybenzoyl)-benzoesäure-n-hexylester stellen eine nicht staubende Form des Esters dar, die sich problemlos in kosmetische Zubereitungen einarbeiten lässt.

Die Erfindung wird durch folgende, die Erfindung jedoch nicht einschränkende Beispiele erläutert.

Der Schmelzpunkt des eingesetzten 2-(4-N,N-Diethylamino-2-hydroxybenzoyl)-benzoesäure-n-hexylesters wurde gemäß Ph.Eur. (European Pharmacopoeia 5.0) gemessen. Es wurde ein Wert von 57°C als Schmelzpunkt ermittelt.

### Versuche zur kontrollierten Kristallisation

### Beispiel 1 - Vergleichsbeispiel

5 kg von aufgeschmolzenem 2-(4-N,N-Diethylamino-2-hydroxybenzoyl)-benzoesäuren-hexylester mit einer Reinheit von mehr als 99% ließ man bei Raumtemperatur abkühlen und stehen, um die Kristallisation der Substanz zu erreichen. Erste Kristalle wurden nach 10 Tagen beobachtet. Nach 2 Monaten war die gesamte Masse des Esters kristallisiert.

### Beispiel 2 - Vergleichsbeispiel

Zu 5 kg von aufgeschmolzenem 2-(4-N,N-Diethylamino-2-hydroxybenzoyl)-benzoesäure-n-hexylester wurden bei etwa 40°C 100 g feine Kristalle von 2-(4-N,N-Diethylamino-2-hydroxybenzoyl)-benzoesäure-n-hexylester (< 100 µm) gegeben. Anschließend ließ man die Schmelze auf Raumtemperatur abkühlen. Erste Kristalle wurden nach 10 Tagen beobachtet. Nach 2 Monaten war die gesamte Masse des Esters kristallisiert.

### Beispiel 3 - Vergleichsbeispiel

5 kg von aufgeschmolzenem 2-(4-N,N-Diethylamino-2-hydroxybenzoyl)-benzoesäuren-hexylester ließ man auf 5°C abkühlen und bei dieser Temperatur stehen, um die Kristallisation der Substanz zu erreichen. Erste Kristalle wurden nach 10 Tagen beobachtet. Nach 2 Monaten bei 5°C war die gesamte Masse des Esters kristallisiert.

### Beispiel 4 - Vergleichsbeispiel

Zu 5 kg von aufgeschmolzenem 2-(4-N,N-Diethylamino-2-hydroxybenzoyl)-benzoesäure-n-hexylester wurden bei etwa 40°C 100 g feine Kristalle von 2-(4-N,N-Diethylamino-2-hydroxybenzoyl)-benzoesäure-n-hexylester (< 100 µm) gegeben. Anschließend ließ man die Schmelze auf Raumtemperatur abkühlen. Erste Kristalle wurden nach 10 Tagen beobachtet. Nach 2 Monaten war die gesamte Masse des Esters kristallisiert.

### Beispiel 5 (erfindungsgemäß)

5 kg von aufgeschmolzenem 2-(4-N,N-Diethylamino-2-hydroxybenzoyl)-benzoesäuren-hexylester wurden in einen 5 l Aluminiumbehälter gefüllt. Mit einem Magnetrührstab (40 mm) wurde die auf Raumtemperatur abkühlende Schmelze für 4 Stunden mit 80 U/min gerührt. Nach 4 Stunden wurden erste Kristalle beobachtet. Vollständige Kristallisation erfolgte innerhalb von 14 Tagen.

### Beispiel 6 (erfindungsgemäß)

5 kg von aufgeschmolzenem 2-(4-N,N-Diethylamino-2-hydroxybenzoyl)-benzoesäuren-hexylester wurden in einen 5 l Aluminiumbehälter gefüllt. Die Schmelze wurde mit einem PTFE-Propellerrührer (60 mm Durchmesser), der durch einen Elektromotor angetrieben wurde, mit einer Rührgeschwindigkeit von 250 U/min bei Raumtemperatur gerührt. Nachdem die Schmelze für 11 Stunden gerührt worden war, war die Viskosität der Schmelze so hoch angestiegen, dass das Rühren nicht fortgeführt werden konnte. Erste Kristalle erschienen nach fünfstündigem Rühren und vollständige Kristallisation erfolgte innerhalb von 24 Stunden.

### Beispiel 7 (erfindungsgemäß)

800 g von aufgeschmolzenem 2-(4-N,N-Diethylamino-2-hydroxybenzoyl)-benzoesäuren-hexylester wurden in einen 1 l Glas-Doppelmantel-Reaktor (HWS) gefüllt. Die Temperatur der Schmelze betrug 60°C. Die Schmelze wurde mit einem PTFE Blattrührer (75 mm Durchmesser) für 6 Stunden mit einer Rührgeschwindigkeit von 350 U/min gerührt. Erste Kristalle wurden nach 4 Stunden beobachtet und die Temperatur der Schmelze betrug zu diesem Zeitpunkt etwa 33°C. Diese Temperatur der Schmelze blieb für einige Zeit konstant. Nach 6 Stunden wurde eine sehr trübe Schmelze beobachtet, deren Temperatur 33°C betrug. Diese trübe, viskose Schmelze wurde aus dem Reaktor in einen Alumiumkristallisationsbehälter umgefüllt. Vollständige Kristallisation erfolgte innerhalb von 24 Stunden.

### Beispiel 8 (erfindungsgemäß)

800 g von aufgeschmolzenem 2-(4-N,N-Diethylamino-2-hydroxybenzoyl)-benzoesäuren-hexylester wurden in einen 1 l Glas-Doppelmantel-Reaktor (HWS) gefüllt, und die Schmelze wurde mit einem PTFE Blattrührer (75 mm Durchmesser) mit einer Rührgeschwindigkeit von 250 U/min gerührt. Nach 2 Stunden wies die Schmelze eine konstant bleibende Temperatur von 28°C auf. Der Trübungspunkt der Schmelze wurde nach zwölfstündigem Rühren erreicht. Vollständige Kristallisation des Esters erfolgte innerhalb von 24 Stunden.

### Beispiel 9 (erfindungsgemäß)

1000 g von aufgeschmolzenem 2-(4-N,N-Diethylamino-2-hydroxybenzoyl)-benzoesäure-n-hexylester wurden als 60°C warme Schmelze in einen 1 l Glas-Doppelmantel-Reaktor (HWS) gefüllt. Die Schmelze kühlte ohne Rühren innerhalb von 2 Stunden auf eine Temperatur von 32°C. Anschließend wurde die Schmelze für 18 Stunden ohne Rühren bei 35°C thermostatisiert gelagert. Die Thermostatisierung wurde abgeschaltet, und die Schmelze wurde mit einem PTFE Propellerrührer (60 mm Durchmesser) für 30 Minuten mit einer Rührgeschwindigkeit von 350 U/min gerührt. Nach zehnminütigem Rühren betrug die Temperatur der Schmelze 33°C. Nach dreißigminütigem Rühren betrug die Temperatur der Schmelze 33,5°C. Die Schmelze wurde trüb und erste Kristalle zeigten sich. Daraufhin wurde die Schmelze in eine Aluminumform umgefüllt. Vollständige Kristallisation des Esters erfolgte innerhalb von 4 Stunden.

### Versuche zur Mahlung

### Beispiel 10 (erfindungsgemäß)

100 g von kristallinem 2-(4-N,N-Diethylamino-2-hydroxybenzoyl)-benzoesäure-n-hexylester aus Beispiel 9 wurden in einer Reibmühle (Alexanderreibe in Lebensmittelausführung mit einem 3-flügeligen Rotor der Firma Alexanderwerk Remscheid) mit einem Sieb der Maschenweite 4,0 x 7,5 mm zerkleinert. 71,2 % des zerkleinerten Materials hatte eine Partikelgröße im Bereich von 0,5 bis 5 mm. Der Feinanteil mit einer Teilchengröße von kleiner als 100 µm wies einen Anteil von 2,5 Gew.-% auf. Dieser Feinanteil wurde durch Sieben bestimmt.

## Patentansprüche

1. Verfahren zur Kristallisation von 2-(4-N,N-Diethylamino-2-hydroxybenzoyl)-benzoesäure-n-hexylester, umfassend die Verfahrensschritte
a) Bereitstellung einer klaren Schmelze von 2-(4-N,N-Diethylamino-2-hydroxybenzoyl)-benzoesäure-n-hexylester bei einer Temperatur oberhalb von 57°C, und
b) Auskristallisation von 2-(4-N,N-Diethylamino-2-hydroxybenzoyl)-benzoesäure-n-hexylester bei einer Temperatur unterhalb von 57°C,
**dadurch gekennzeichnet, dass** die klare Schmelze aus Verfahrenschritt a) bei einer Temperatur unterhalb von 57°C so lange gerührt wird, bis eine Trübung auftritt, bevor Verfahrenschritt b) ausgeführt wird.

2. Verfahren zur Kristallisation von 2-(4-N,N-Diethylamino-2-hydroxybenzoyl)-benzoesäure-n-hexylester gemäß Anspruch 1, wobei die Schmelze von 2-(4-N,N-Diethylamino-2-hydroxybenzoyl)-benzoesäure-n-hexylester in Verfahrensschritt a) eine Reinheit von mindestens 98 Gew.-% aufweist.

3. Verfahren gemäß Anspruch 1 oder 2, wobei die in Verfahrensschritt a) hergestellte Schmelze bei einer Temperatur zwischen 25 und 40°C bis zur Trübung gerührt wird.

4. Verfahren gemäß einem der Ansprüche 1 bis 3, wobei Verfahrensschritt b) bei einer Temperatur von 15 bis 35°C durchgeführt wird.

5. Verfahren gemäß einem der Ansprüche 1 bis 4, wobei die Schmelze mit einem Rührer, der einen Durchmesser von 2 bis 20 cm aufweist, bei 200 bis 500 U/min gerührt wird.

6. Verfahren zur Herstellung von rieselfähigen oder schüttbaren Teilchen von kristallinem 2-(4-N,N-Diethylamino-2-hydroxybenzoyl)-benzoesäure-n-hexylester mit einem mittleren Teilchendurchmesser von 10 µm bis 22 cm, umfassend die Schritte
a') Bereitstellung einer klaren Schmelze von 2-(4-N,N-Diethylamino-2-hydroxybenzoyl)-benzoesäure-n-hexylester bei einer Temperatur oberhalb von 57°C,
b') Rühren der Schmelze aus Verfahrensschritt a') bei einer Temperatur unterhalb von 57°C bis eine Trübung auftritt,
c') Abfüllen der trüben Schmelze in eine Form,
d') Auskristallisation der abgefüllten Schmelze von 2-(4-N,N-Diethylamino-2-hydroxybenzoyl)-benzoesäure-n-hexylester bei einer Temperatur unterhalb von 57°C in der Form,
e') Entnahme des auskristallisierten 2-(4-N,N-Diethylamino-2-hydroxybenzoyl)-benzoesäure-n-hexylesters aus der Form und optional
f') Zerkleinern des kristallinen 2-(4-N,N-Diethylamino-2-hydroxybenzoyl)-benzoesäure-n-hexylesters aus Verfahrensschritt e') auf die gewünschte Teilchengröße.

7. Verfahren gemäß Anspruch 6, wobei die Schmelze von 2-(4-N,N-Diethylamino-2-hydroxybenzoyl)-benzoesäure-n-hexylester in Verfahrensschritt a') eine Reinheit von mindestens 98 Gew.-% aufweist.

8. Verfahren gemäß Anspruch 6 oder 7, wobei Verfahrensschritt b') bei einer Temperatur zwischen 25 und 40°C durchgeführt wird.

9. Verfahren gemäß einem der Ansprüche 6 bis 8, wobei Verfahrensschritt d') bei einer Temperatur von 15 bis 35°C durchgeführt wird.

10. Rieselfähige oder schüttbare Teilchen von kristallinem 2-(4-N,N-Diethylamino-2-hydroxybenzoyl)-benzoesäure-n-hexylester mit einem mittleren Teilchendurchmesser von 0,1 bis 5 mm, einer Schüttdichte größer als 0,5 g/ml und einer Reinheit von mindestens 98 Gew.-%.

11. Verwendung der rieselfähigen oder schüttbaren Teilchen von kristallinem 2-(4-N,N-Diethylamino-2-hydroxybenzoyl)-benzoesäure-n-hexylester gemäß Anspruch 10 als UV-Filter oder als Radikalfänger in kosmetischen und dermatologischen Zubereitungen oder als Produktschutz.

## Claims

1. A method for the crystallization of n-hexyl 2-(4-N,N-diethylamino-2-hydroxybenzoyl)benzoate, comprising the steps
a) providing a clear melt of n-hexyl 2-(4-N,N-diethylamino-2-hydroxybenzoyl)-benzoate at a temperature above 57°C, and
b) crystallizing out n-hexyl 2-(4-N,N-diethylamino-2-hydroxybenzoyl)benzoate at a temperature below 57°C,
wherein the clear melt from process step a) is stirred at a temperature below 57°C until opacity arises, before process step b) is carried out.

2. The method for the crystallization of n-hexyl 2-(4-N,N-diethylamino-2-hydroxybenzoyl)benzoate according to claim 1, where the melt of n-hexyl 2-(4-N,N-diethylamino-2-hydroxybenzoyl)benzoate in process step a) has a purity of at least 98% by weight.

3. The method according to claim 1 or 2, where the melt prepared in process step a) is stirred at a temperature between 25 and 40°C until opaque.

4. The method according to any of claims 1 to 3, where process step b) is carried out at a temperature of from 15 to 35°C.

5. The method according to any of claims 1 to 4, where the melt is stirred with a stirrer which has a diameter of from 2 to 20 cm at 200 to 500 rpm.

6. A method for the production of pourable or flowable particles of crystalline n-hexyl 2-(4-N,N-diethylamino-2-hydroxybenzoyl)benzoate having an average particle diameter of from 10 µm to 22 cm, comprising the steps
a') providing a clear melt of n-hexyl 2-(4-N,N-diethylamino-2-hydroxybenzoyl)-benzoate at a temperature above 57°C,
b') stirring the melt from process step a') at a temperature below 57°C until opacity arises,
c') transferring the opaque melt to a mold,
d') crystallizing out the transferred melt of n-hexyl 2-(4-N,N-diethylamino-2-hydroxybenzoyl)benzoate at a temperature below 57°C in the mold,
e') removing the crystallized-out n-hexyl 2-(4-N,N-diethylamino-2-hydroxybenzoyl)benzoate from the mold and optionally
f') comminuting the crystalline n-hexyl 2-(4-N,N-diethylamino-2-hydroxybenzoyl)benzoate from process step e') to the desired particle size.

7. The method according to claim 6, where the melt of n-hexyl 2-(4-N,N-diethylamino-2-hydroxybenzoyl)benzoate in process step a') has a purity of at least 98% by weight.

8. The method according to claim 6 or 7, where process step b') is carried out at a temperature between 25 and 40°C.

9. The method according to any of claims 6 to 8, where process step d') is carried out at a temperature of from 15 to 35°C.

10. A pourable or flowable particle of crystalline n-hexyl 2-(4-N,N-diethylamino-2-hydroxybenzoyl)benzoate with an average particle diameter of from 0.1 to 5 mm, a bulk density greater than 0.5 g/ml and a purity of at least 98% by weight.

11. The use of the pourable or flowable particles of crystalline n-hexyl 2-(4-N,N-diethylamino-2-hydroxybenzoyl)benzoate according to claim 10 as UV filter or as free-radical scavenger in cosmetic and dermatological preparations or as product protection.

## Revendications

1. Procédé de cristallisation d'ester n-hexylique de l'acide 2-(4-N,N-diéthylamino-2-hydroxybenzoyl)-benzoïque, comprenant les étapes de procédé suivantes :
a) la préparation d'une masse fondue transparente d'ester n-hexylique de l'acide 2-(4-N,N-diéthylamino-2-hydroxybenzoyl)-benzoïque à une température supérieure à 57 °C et
b) la cristallisation de l'ester n-hexylique de l'acide 2-(4-N,N-diéthylamino-2-hydroxybenzoyl)-benzoïque à une température inférieure à 57 °C,
**caractérisé en ce que** la masse fondue transparente de l'étape de procédé a) est agitée à une température inférieure à 57 °C jusqu'à ce qu'un trouble se produise avant la réalisation de l'étape de procédé b).

2. Procédé de cristallisation d'ester n-hexylique de l'acide 2-(4-N,N-diéthylamino-2-hydroxybenzoyl)-benzoïque selon la revendication 1, dans lequel la masse fondue d'ester n-hexylique de l'acide 2-(4-N,N-diéthylamino-2-hydroxybenzoyl)-benzoïque à l'étape de procédé a) présente une pureté d'au moins 98 % en poids.

3. Procédé selon la revendication 1 ou 2, dans lequel la masse fondue fabriquée à l'étape de procédé a) est agitée à une température comprise entre 25 et 40 °C jusqu'au trouble.

4. Procédé selon l'une quelconque des revendications 1 à 3, dans lequel l'étape de procédé b) est réalisée à une température de 15 à 35 °C.

5. Procédé selon l'une quelconque des revendications 1 à 4, dans lequel la masse fondue est agitée avec un agitateur présentant un diamètre de 2 à 20 cm, à 200 à 500 tours/min.

6. Procédé de fabrication de particules fluides ou versables d'ester n-hexylique de l'acide 2-(4-N,N-diéthylamino-2-hydroxybenzoyl)-benzoïque cristallin ayant un diamètre de particule moyen de 10 µm à 22 cm, comprenant les étapes suivantes :
a') la préparation d'une masse fondue transparente d'ester n-hexylique de l'acide 2-(4-N,N-diéthylamino-2-hydroxybenzoyl)-benzoïque à une température supérieure à 57 °C,
b') l'agitation de la masse fondue de l'étape de procédé a') à une température inférieure à 57 °C jusqu'à ce qu'un trouble se produise,
c') le remplissage d'un moule avec la masse fondue trouble,
d') la cristallisation de la masse fondue remplie d'ester n-hexylique de l'acide 2-(4-N,N-diéthylamino-2-hydroxybenzoyl)-benzoïque à une température inférieure à 57 °C dans le moule,
e') l'extraction de l'ester n-hexylique de l'acide 2-(4-N,N-diéthylamino-2-hydroxybenzoyl)-benzoïque cristallisé du moule et éventuellement
f') le broyage de l'ester n-hexylique de l'acide 2-(4-N,N-diéthylamino-2-hydroxybenzoyl)-benzoïque cristallin de l'étape de procédé e') à la taille de particule souhaitée.

7. Procédé selon la revendication 6, dans lequel la masse fondue d'ester n-hexylique de l'acide 2-(4-N,N-diéthylamino-2-hydroxybenzoyl)-benzoïque à l'étape de procédé a') présente une pureté d'au moins 98 % en poids.

8. Procédé selon la revendication 6 ou 7, dans lequel l'étape de procédé b') est réalisée à une température comprise entre 25 et 40 °C.

9. Procédé selon l'une quelconque des revendications 6 à 8, dans lequel l'étape de procédé d') est réalisée à une température de 15 à 35 °C.

10. Particules fluides ou versables d'ester n-hexylique de l'acide 2-(4-N,N-diéthylamino-2-hydroxybenzoyl)-benzoïque cristallin ayant un diamètre de particule moyen de 0,1 à 5 mm, une densité apparente supérieure à 0,5 g/ml et une pureté d'au moins 98 % en poids.

11. Utilisation des particules fluides ou versables d'ester n-hexylique de l'acide 2-(4-N,N-diéthylamino-2-hydroxybenzoyl)-benzoïque cristallin selon la revendication 10 en tant que filtre UV ou capteur de radicaux dans des préparations cosmétiques et dermatologiques ou en tant que protection de produit.
